# EUROPÄISCHE PATENTSCHRIFT

(11) **EP 4 555 984 B1**
(45) Veröffentlichungstag und Bekanntmachung des Hinweises auf die Patenterteilung: **22.07.2026**
(21) Anmeldenummer: 24214096.0
(22) Anmeldetag: 20.11.2024
(51) Int. Cl.: A61F 11/00

(54) **WIEDERVER-WENDBARES OHRENREINIGUNGSSTÄBCHEN MIT OFFENEM ABRAUMKORB**
RE-USABLE EAR CLEANING STICK WITH AN OPEN BASKET
BÂTON DE NETTOYAGE D'OREILLES RÉUTILISABLE AVEC PANIER OUVERT

(30) Priorität: 20.11.2023 DE 202023002416 U
(43) Veröffentlichungstag der Anmeldung: 21.05.2025
(73) Patentinhaber: Velev, Dimo, 80802 München (DE)
(72) Erfinder: Velev, Dimo, 80802 München (DE)
(74) Vertreter: Kiwit, Benedikt

(56) Entgegenhaltungen:
- WO-A1-2022/217741
- FR-A1- 2 600 883
- US-A1- 2003 135 228
- US-A1- 2012 296 355

## Beschreibung

Das Problem:
Es gibt bereits verschiedene mechanische (ohne Wasser oder spezielle Flüssigkeiten) Instrumente zur Reinigung des Ohres beziehungsweise zur Entfernung von überschüssigem Ohrenschmalz (Cerumen) aus dem Gehörgang. Die meisten haben eine kompakte, geschlossene Form. Stellvertretend sei hier auf das bekannteste Gerät, das Wattestäbchen ("Q-Tipp") verwiesen, welches den großen Nachteil hat, dass es aufgrund seines kompakten Wattekopfes beim Einführen in den Gehörgang Ohrenschmalz oft tiefer in den Gehörgang schiebt, was zu einer weiteren Verpfropfung führen kann. Andere Lösungen, zum Beispiel aus Metall, bergen die Gefahr einer Verletzung des empfindlichen Gehörgangs.

Aus der WO 2022/217741 A1 ist ein Gerät bekannt, bei der in einem Griff ein Antrieb vorgesehen ist, mit dem ein aufsetzbares Reinigungsstäbchen gedreht werden kann. Das Reinigungsstäbchen weist ein Röhrchen auf, an dessen in das Ohr einführbaren Ende ein asymmetrisch ausgebildeter Abraumkorb vorhanden ist, wobei der Abraumkorb aus einer Mehrzahl von nach außen gebogenen Streben gebildet ist. Auf der von dem Röhrchen abgewandten Seite münden die einzelnen Streben in einen asymmetrischen Ring.

Zur Vermeidung, dass ein Pfropfen während des Reinigungsvorgangs im Gehörgang weiter in Richtung Trommelfell geschoben wird, sind in der Vergangenheit bereits Anstrengungen unternommen worden, um Geometrien des Reinigungsstäbchens zu entwickeln, mit denen dies verhindert werden kann. So ist beispielsweise aus der CH 700 707 B1 eine Lösung bekannt, bei der das Reinigungsstäbchen zumindest in dem Bereich, mit dem es in den Gehörgang eingeführt wird, hohl ausgebildet ist. Am Außenumfang dieses Abschnitts ist eine Wendel angebracht, sodass durch Drehen Cerumen aus dem Gehörgang entfernt werden kann. Da die Stirnfläche des Reinigungsstäbchens nicht geschlossen ausgeführt ist, wird verhindert, dass sich Cerumen vor dem Reinigungsstäbchens beim Einführen staut und so in Richtung des Trommelfells geschoben wird. Nachteilig ist hierbei allerdings, dass zum einen Cerumen nur aus dem äußeren Bereich des Gehörgangs entfernt werden kann, nicht aber Cerumen, welches sich bereits Bereich vor dem Trommelfell angelagert hat. Teil hiervon werden unglücklicherweise auch in das Innere des hohlen Abschnitts geschoben, aus dem es schwierig wieder zu entfernen ist. Aufgrund der kleinen Abmessungen solcher Reinigungsstäbchen ist es kaum möglich, eine hygienisch befriedigende Lösung für deren Reinigung zu finden. Eine Wiederverwendung des vorgeschlagenen Reinigungsstäbchens ist daher kaum denkbar und im Hinblick auf einen verbesserten Umweltschutz ist die Verwendung solcher in der Regel aus Kunststoff gefertigten Reinigungsstäbchens als Wegwerfartikel kritisch zu sehen.

Eine weitere Lösung ist aus der DE 600 05740 T2 bekannt, in der insbesondere auch die Herstellung eines Reinigungsstäbchens beschrieben ist. Bei der dort vorgeschlagene Lösung ist wenigstens an einem Ende eines Griffabschnitts eine Anordnung aus zwei Halbschalen ausgebildet, welche vor der Verwendung des Reinigungsstäbchens zusammengeklappt werden müssen, sodass ein Reinigungselement gebildet wird, das ein Hohlvolumen bildet, dessen äußere Schale mit Schlitzen zur Aufnahme von Cerumen versehen ist. Durch die im Hinblick auf die Fertigung optimierte Lösung ist dieses Hohlvolumen aus den beiden Halbschalen so gefertigt, dass die beiden Halbschalen in den Bereich, der schließlich das von dem Griff abgewandte Ende bildet, miteinander verbunden sind. Zwar soll durch Drehen des Reinigungsstäbchens mit dem Hohlvolumen durch die Schlitze eine Reinigung des Gehörgangs möglich sein, jedoch besteht weiterhin das Problem, dass die in Einführungsrichtung gelegene Vorderseite des Hohlvolumens eine geschlossene Fläche bildet. Damit wird wiederum Cerumen nachteilig in Richtung des Trommelfells transportiert. Die vorgesehenen Schlitze in dem Volumen sind zudem so klein, dass ein Entfernen von Cerumen aus dem Gehörgang zwar möglich ist, jedoch auch eine Reinigung des Reinigungsstäbchens schwierig sein dürfte. Eine Wiederverwendung des vorgeschlagenen Reinigungsstäbchens scheint daher aus hygienischen Gründen ebenfalls nicht möglich zu sein. Die mehrteilige Ausbildung des Hohlvolumens birgt zudem das Risiko, dass sich die Arretierung zum Fixieren der zusammen beklagten Halbschale im Gehörgang löst und es beim Herausziehen des Reinigungsstäbchens somit zu Verletzungen kommen kann.

Die funktionelle Lösung:
Um überschüssiges Ohrenschmalz schonend, ohne Verletzung des Gehörganges zu entfernen, Verpfropfungen zu vermeiden oder bestehende abzubauen, wurde das wiederverwendbare Ohrenreinigungsstäbchen mit einem speziellen offenen Abraumkorb entwickelt.

Das gesamte Stäbchen (inklusive Abraumkorb am Kopfende) besteht aus einem elastischen, nachgiebigen Kunststoff (z.B. Silikon oder Gummi), der eine gewisse Stabilität aufweist, und somit schonend in den Gehörgang eingeführt werden kann. Den Kopf des Stäbchens bildet ein speziell entwickelter Abraumkorb, welcher aufgrund seiner offenen Konstruktion in den Gehörgang eingeführt wird, ohne dass Ohrenschmalz tiefer in den Gehörgang geschoben wird. Durch Einführen des Ohrenstäbchens in den Gehörgang wird überschüssiges Ohrenschmalz vom Abraumkorb aufgenommen. Durch die anschließende Drehung des Stäbchens wird durch die Streben des Korbes weiteres Ohrenschmalz von der Wand des Gehörganges aufgenommen und das so aufgenommene überschüssige Cerumen im Abraumkorb verdichtet. Dieses kann dann mit dem Stäbchen rausgezogen und schonend aus dem Gehörgang entfernt werden.

Danach kann das Stäbchen gereinigt (z.B. unter laufenden Wasser) und später wiederverwendet werden.

Das technisch Neue:
Diese Entwicklung ähnelt in ihren Abmessungen bestehenden Geräten (z.B. Wattestäbchen) unterscheidet sich aber grundlegend durch den neuentwickelten speziellen offenen Abraumkorb, welcher überschüssiges Ohrenschmalz aufnimmt und verdichtet, ohne Ohrenschmalz tiefer in den Gehörgang zu drücken.

Durch die neuartige Konstruktionsform in Kombination mit der Nachgiebigkeit des Materials des Ohrenstäbchens wird zudem Verletzungen des Gehörganges vorgebeugt.

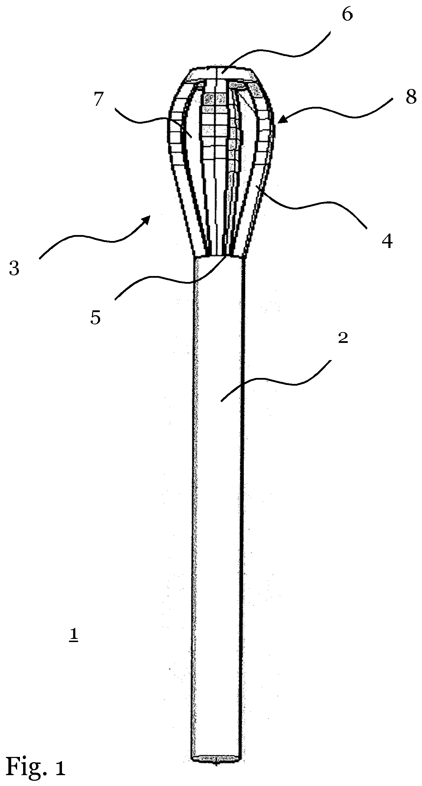

Das Ohrenstäbchen kann nach jeder Anwendung gereinigt und dann wiederverwendet werden. Das gesamte Stäbchen, sowie der Abraumkorb alleine, sind in der Figur 1 dargestellt.

Die technische Lösung gemäß einer bevorzugten Ausführungsform ist in Figur 1 dargestellt und wird nachfolgend näher erläutert.

Das bevorzugte Ausführungsbeispiel eines erfindungsgemäßen Reinigungsstäbchens 1 weist einen Stäbchengriff 2 auf, an dem, an wenigstens einem Ende, ein Abraumkorb 3 zum Entfernen von Cerumen aus dem Gehörgang angeordnet ist. Der Abraumkorb 3 wird durch eine Mehrzahl von Streben 4 gebildet, die auf einer Seite mit dem Stäbchengriff 2 verbunden sind und an ihren jeweils von dem Stäbchengriff 2 abgewandten Enden mit einem Ring 6 verbunden sind.

Der Ring 6 ist kreisförmig ausgebildet und so orientiert, dass seine Mittelachse mit der Mittelachse des Stäbchengriffs 2 zusammenfällt. Die in Einführrichtung orientierte geschlossene Fläche während des Reinigungsvorgangs wird daher lediglich durch den schmalen Ring 6 gebildet, sodass über den offenen Teil des Rings 6 Cerumen in das Innere des Abraumkorbs 3 eindringen kann.

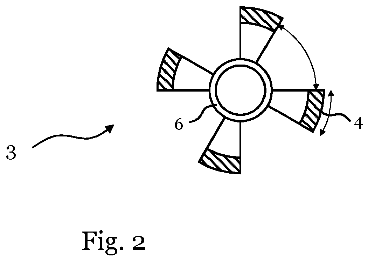

Die Streben 4 sind zwischen dem Stäbchengriff und dem Ring 6 nach außen gewölbt, sodass vom Stäbchengriff 2 bis zum Ring 6 eine etwa keulenförmige Geometrie des Abraumkorbs 3 entsteht. Der Abraumkorb 3 ist vorzugsweise symmetrisch zur Längsachse des gesamten Stäbchens 1. Zumindest im Bereich der größten Ausdehnung der Keule in seitlicher Richtung, die in der Figur 1 mit dem Bezugszeichen 8 versehen ist, ist, in Umfangsrichtung betrachtet, der Abstand zwischen den Streben 4 größer als die Ausdehnung der Streben 4 in Umfangsrichtung. Dies ist in der Figur 2 dargestellt, die einen Schnitt durch den Abraumkorb 3 mit Blick auf den Ring 6 zeigt. Die beiden Pfeile zeigen die Ausdehnungen des Abstands zwischen den Streben 4 und die Ausdehnung der Streben 4 selbst in Umfangsrichtung an der größten seitlichen Ausdehnung des Abtraumkorbs 3. Auf diese Weise wird sichergestellt, dass zumindest im Abschnitt mit der größten seitlichen Ausdehnung des Abraumkorbs 3 ein einfacher Zugang zum Innenraum des Abraumkorbs 3 vorhanden ist. Dies ist nicht nur vorteilhaft im Hinblick auf eine leichte Aufnahme von Cerumen in das Innere des Abraumkorbs 3, sondern auch im Hinblick auf eine Reinigung nach Abschluss des Reinigungsvorgangs.

Durch den verhältnismäßig großen, offenen Anteil des Abraumkorbs 3 wird einerseits gewährleistet, dass das Ausspülen des Abraumkorbs 3 mit Wasser effizient und einfach möglich ist, andererseits aber auch, dass eine relativ leichte Verformung des Abraumkorbs 3 möglich ist, was ein Anpassen der Form während des Reinigungsvorgangs an den Gehörgang ermöglicht.

Die keulenförmige Geometrie des Abraumkorbs 3 kommt, wie es in der Figur 1 dargestellt ist, dadurch zustande, dass sich die seitlichen Abmessungen des Abraumkorbs 3 aufgrund der Krümmung der Streben 4 ausgehend von ihrer größten Ausdehnung in mit 8 bezeichneten Bereich in Richtung zu dem Ring 6 hin schneller reduzieren als in Richtung zu dem Stäbchengriff 2 hin. Damit wird die Reinigungswirkung zusätzlich verbessert, da beim Herausziehen, vorzugsweise mit einer Drehbewegung, das im Inneren des Abraumkorbs 3 eingeschlossene Cerumen leichter verblockt und damit das Wiederaustreten durch den Ring 6 verhindert werden kann. Zwar ist in der Darstellung der Figur 1 eine Trennlinie 5 zwischen dem Stäbchengriff 2 und den Streben 4 zu erkennen, diese ergibt sich jedoch nur aufgrund des Knicks der an dieser Stelle zwischen den Stäbchengriff 2 und den Streben 4 ausgebildet ist. Tatsächlich sind in der dargestellten bevorzugten Ausführungsform die Streben 4 und der Stäbchengriff 2 einstückig.

Das Vorsehen des Rings 6 am stirnseitigen Ende des Stäbchens 1 hat außerdem den Vorteil, dass zur Herstellung, beispielsweise im Spritzgussverfahren, ein Dorn eingeführt werden kann, der mit mehreren Elementen, die radial zugeführt werden, eine Form für den Abraumkorbs 3 bildet. Gemäß einer alternativen Ausführungsform wäre es auch denkbar, dass der Stäbchengriff 2 als hohles Rohr ausgebildet ist, und freie Enden der Streben 4, welche nur einstückig mit dem Ring 6 verbunden sind, in das Innere des Stäbchengriff 2 eingeführt werden und erst dann dauerhaft mit diesem verbunden werden.

Der Ring 6 hat vorzugsweise einen äußeren Durchmesser von 0,4 bis 0,5 cm und ist im dargestellten Ausführungsbeispiel durch genau vier nach außen gebogene Streben von jeweils 1,5 bis 1,8 cm Länge mit dem Stäbchengriff 2 verbunden. Die breite des Rings in radialer Richtung ist möglichst klein, damit die geschlossene Stirnfläche ebenfalls möglichst klein ist. Der innere Durchmesser kann beispielsweise etwa einen Millimeter kleiner gewählt werden als der äußere Durchmesser. Die vier Strebern 4 gehen in diesen über. Grundsätzlich ist jedoch auch eine andere Anzahl an Streben 4 denkbar. Es ist auch möglich, dass die Streben unterschiedliche Querschnitte besitzen.

Stäbchengriff 2 und Abraumkorb 3 sind eine verbundene Einheit aus identischem, leicht elastischem Material (z.B. Silikon oder Gummi), welches sich dem leicht gebogenen Verlauf des Gehörgangs anpasst.

Das Stäbchen 1 ist einteilig und besteht durchgängig aus dem gleichen Material.

Es hat je nach Ausführung eine Gesamtlänge von 5,5 bis 7 cm.

## Patentansprüche

1. Wiederverwendbares Ohrenreinigungsstäbchen (1) mit integriertem offenen Abraumkorb (3), wobei der Abraumkorb (3) aus einer Mehrzahl von nach außen gebogenen Streben (4) und einem kreisförmigen Ring (6) gebildet wird, wobei die Streben (4) mit einem Stäbchengriff (2) und an ihrem anderen Ende mit dem Ring (6) verbunden sind, dessen Mittelachse mit der Mittelachse des Stäbchengriffs (2) zusammenfällt.

2. Wiederverwendbares Ohrenreinigungsstäbchen (1), wobei ein Abstand zwischen den Streben (4) in Umfangsrichtung des Abraumkorbs (3) zumindest im Bereich der größten seitlichen Ausdehnung des Abraumkorbs (3) größer oder gleich einer Ausdehnung der Streben (4) im Bereich der größten seitlichen Ausdehnung des Abraumkorbs (3) entlang des Umfangs ist.

3. Wiederverwendbares Ohrenreinigungsstäbchen (1), wobei das Ohrenreinigungsstäbchen (2) aus einem elastischen Material, insbesondere Silikon oder Gummi, gefertigt ist.

## Claims

1. Re-usable ear cleaning stick (1) with an integrated open debris collection basket (3),
wherein the debris collection basket (3) is formed from a plurality of outwardly curved struts (4) and a circular ring (6), wherein the struts (4) are connected at one end to a stick handle (2) and at their other end to the ring (6), the central axis of which coincides with the central axis of the stick handle (2).

2. Re-usable ear cleaning stick (1), wherein a distance between the struts (4) in the circumferential direction of the debris collection basket (3), at least in the region of the greatest lateral extent of the debris collection basket (3), is greater than or equal to an extent of the struts (4) in the region of the greatest lateral extent of the debris collection basket (3) along the circumference.

3. Re-usable ear cleaning stick (1), wherein the ear cleaning stick (2) is made of an elastic material, in particular silicone or rubber.

## Revendications

1. Bâtonnet de nettoyage d'oreilles réutilisable (1) avec récipient à déchets (3) ouvert intégré, dans lequel le récipient à déchets (3) est formé d'une pluralité de barres (4) courbées vers l'extérieur et d'un anneau (6) circulaire, dans lequel les barres (4) sont reliées à une poignée de bâtonnet (2) et à leur autre extrémité à l'anneau (6) dont l'axe central coïncide avec l'axe central de la poignée de bâtonnet (2).

2. Bâtonnet de nettoyage d'oreilles réutilisable (1), dans lequel une distance entre les barres (4) dans la direction circonférentielle du récipient à déchets (3) est au moins dans la zone de la plus grande extension latérale du récipient à déchets (3) supérieure ou égale à une extension des barres (4) dans la zone de la plus grande extension latérale du récipient à déchets (3) le long de la circonférence.

3. Bâtonnet de nettoyage d'oreilles réutilisable (1), dans lequel le bâtonnet de nettoyage d'oreilles (2) est fabriqué dans un matériau élastique, en particulier du silicone ou du caoutchouc.
